(19) Europäisches Patentamt
European Patent Office
Office européen des brevets

(11) Numéro de publication : **0 099 841**
**B1**

# (12) FASCICULE DE BREVET EUROPÉEN

(45) Date de publication du fascicule du brevet : **02.01.86**

(51) Int. Cl.$^4$ : **C 07 C 51/14**, C 07 C 57/03, C 07 C 67/38

(21) Numéro de dépôt : **83420116.2**

(22) Date de dépôt : **07.07.83**

(54) **Procédé de préparation d'acides carboxyliques bêta, gamma-insaturés.**

(30) Priorité : **09.07.82 FR 8212231**

(43) Date de publication de la demande :
**01.02.84 Bulletin 84/05**

(45) Mention de la délivrance du brevet :
**02.01.86 Bulletin 86/01**

(84) Etats contractants désignés :
**AT BE CH DE FR GB IT LI LU NL SE**

(56) Documents cités :
**DE-C- 805 641**
**FR-A- 2 274 595**
**FR-A- 2 291 962**
**FR-A- 2 498 594**
**CHEMICAL ABSTRACTS, vol. 92, no. 3, 21 janvier 1980, page 646, no. 22078h, Columbus, Ohio, USA**

(73) Titulaire : **RHONE-POULENC CHIMIE DE BASE**
**25, quai Paul Doumer**
**F-92408 Courbevoie (FR)**

(72) Inventeur : **Jenck, Jean**
**4, avenue de la Paix**
**F-68490 Chalampe (FR)**

(74) Mandataire : **Varnière-Grange, Monique et al**
**RHONE-POULENC RECHERCHES Service Brevets Chimie et Polymères Centre de Recherches de Saint-Fons 85, avenue des Frères Perret B.P. 62**
**F-69192 St-Fons Cedex (FR)**

**EP 0 099 841 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un procédé de préparation d'acides carboxyliques β,γ-insaturés, par réaction de diènes conjugués et du monoxyde de carbone en présence d'eau.

Il est connu d'après le brevet français 1 406 194 de préparer l'acide pentène-3 oïque par réaction entre le butadiène, le monoxyde de carbone et l'eau, en présence de dichlorure de bis-triphénylphosphine-palladium et d'acide chlorhydrique à une température de l'ordre de 120 à 140 °C et sous une pression de l'ordre de 700 bars.

Néanmoins, malgré les hautes pressions utilisées, ce procédé est peu efficace et la sélectivité en acide recherché n'est pas satisfaisante.

Il est par ailleurs connu d'après le brevet français 1 476 301 de préparer l'acide pentène-3 oïque par réaction entre le butadiène, le monoxyde de carbone et l'eau, en présence de palladium, d'acide chlorhydrique et d'oxygène à une température de 100 °C et sous une pression de l'ordre de 700 bars. Si dans ces conditions on obtient une quantité appréciable d'acide pentène-3 oïque, cette méthode n'est pas satisfaisante, notamment en raison de l'utilisation d'un mélange de monoxyde de carbone et d'oxygène, dangereux et de l'accroissement des risques de corrosion lié à la présence simultanée dans le milieu réactionnel d'eau, d'acide chlorhydrique et d'un oxydant. Par ailleurs, malgré les hautes pressions mises en œuvre, ce procédé reste peu efficace et la sélectivité en acide recherché n'est pas satisfaisante.

La réaction précitée (carbonylation de diènes conjugués en présence d'eau) est par ailleurs décrite dans le brevet américain 3 509 209. Cette réaction est conduite en présence de palladium et d'acide chlorhydrique et, le cas échéant, de composés du fer. Toutefois cette technique n'est pas satisfaisante en raison, notamment du faible taux de conversion du diène observé et de la faible sélectivité en acide recherché.

La Demanderesse a par ailleurs constaté que lorsque l'on fait réagir du butadiène, du monoxyde de carbone et de l'eau, en présence de chlorure de palladium (II) et d'acide chlorhydrique, le complexe catalytique n'est pas stable : il se dégrade en granules de palladium métallique inactif.

Il a maintenant été trouvé un nouveau procédé de préparation d'acides carboxyliques β,γ-insaturés par carbonylation de diènes conjugués en présence d'eau permettant d'obvier aux inconvénients précités et notamment de carbonyler efficacement et sélectivement les diènes conjugués en acides recherchés, dans des conditions de température et de pression relativement douces, le complexe catalytique ayant par ailleurs une stabilité satisfaisante.

La présente invention a donc pour objet un procédé de préparation d'acides carboxyliques β,γ-insaturés, par carbonylation d'un diène conjugué en présence d'eau, d'un hydracide halogéné et d'un catalyseur au palladium, à une température supérieure à 60 °C et sous une pression totale (en température) supérieure à 50 bars, caractérisé en ce que :

a) l'opération de carbonylation est réalisée en outre en présence d'un sel d'onium quaternaire d'un élément du groupe VB choisi parmi l'azote, le phosphore et l'arsenic, ledit élément étant quadricoordiné à des atomes de carbone et ledit sel présentant un anion choisi parmi les bases « dures » ou « intermédiaires » du groupe constitué par les anions : $PO_4^{3-}$, $HPO_4^{2-}$, $H_2PO_4^-$, $CH_3SO_3^-$, Ph-$SO_3^-$, (Ph représentant un radical phényle), $NO_3^-$, $SO_4^{2-}$, $PF_6^-$, $Cl^-$, ou $Br^-$,

b) le catalyseur au palladium est constitué par :

— du palladium métallique éventuellement déposé sur un support,

— un oxyde de palladium,

— un sel ou un complexe π-allylique de palladium dont l'anion coordiné au cation palladium est une base « dure » ou « intermédiaire » du groupe constitué par les anions carboxylates, $SO_4^{2-}$, $NO_3^-$, acétylacétonate, chlorure et bromure, ou

— un complexe du palladium zéro comprenant des ligands organiques ne contenant pas d'éléments du groupe VB.

[Par base « dure » ou « intermédiaire », on entend tout anion répondant à la définition classique donnée par R. PEARSON dans J. Chem. Ed., *45,* 581-7 (1968), les termes « dure » et « intermédiaire » ayant respectivement la signification de « hard » et de « borderline » utilisés dans cette référence.]

Par cation onium quaternaire dont l'élément du groupe VB est quadricoordiné à des atomes de carbone, on entend des cations formés à partir d'azote, de phosphore ou d'arsenic et de quatre groupements hydrocarbonés monovalents, identiques ou différents, dont la valence libre est portée par un atome de carbone, chaque groupement étant relié à l'élément précité par ladite valence libre, deux quelconques de ces groupements pouvant par ailleurs former ensemble un radical unique divalent.

Pour une bonne réalisation du procédé de l'invention, le sel d'onium quaternaire présente un cation onium quaternaire répondant à l'une des formules I à III suivantes :

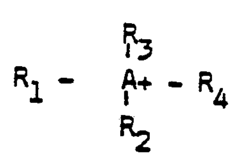

(I)

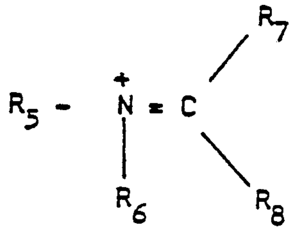

(II)

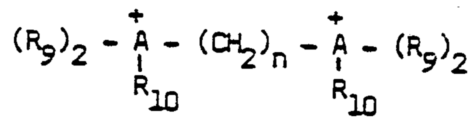

(III)

dans lesquelles :

— A représente un atome d'azote, de phosphore ou d'arsenic,

— $R_1$, $R_2$, $R_3$, $R_4$ sont identiques ou différents et représentent :

— un radical alkyle linéaire ou ramifié contenant de 1 à 16 atomes de carbone, éventuellement substitué par un groupe phényle, hydroxy, halogéno, nitro, alcoxy ou alcoxycarbonyle, le groupement alcoxy renfermant au maximum 4 atomes de carbone ;

— un radical alcényle linéaire ou ramifié contenant de 2 à 12 atomes de carbone, de préférence de 4 à 8 atomes de carbone, et tout particulièrement un radical alcényle dérivé du diène conjugué mis en œuvre ;

— un radical aryle contenant de 6 à 10 atomes de carbone, éventuellement substitué par un ou des radicaux alkyles contenant de 1 à 4 atomes de carbone, alcoxy, alcoxycarbonyle, le groupement alcoxy renfermant au maximum 4 atomes de carbone ou halogéno ;

— deux desdits radicaux $R_1$ à $R_4$ pouvant former ensemble un radical alkylène, alcénylène ou alcadiènylène, linéaire ou ramifié, contenant de 3 à 6 atomes de carbone ;

— $R_5$, $R_6$, $R_7$, $R_8$ sont identiques ou différents et représentent :

— un radical alkyle linéaire ou ramifié contenant de 1 à 4 atomes de carbone ;

— les radicaux $R_7$ et $R_8$ pouvant former ensemble un radical alkylène contenant de 3 à 6 atomes de carbone ;

— les radicaux $R_6$ et $R_7$ ou $R_6$ et $R_8$ pouvant former ensemble un radical alkylène, alcénylène ou alcadiènylène, contenant 4 atomes de carbone et constituant avec l'atome d'azote un hétérocycle azoté ;

— $R_9$ représente un radical alkyle linéaire ou ramifié contenant de 1 à 4 atomes de carbone ou un radical phényle ;

— $R_{10}$ représente :

— un radical alkyle linéaire ou ramifié contenant de 1 à 4 atomes de carbone, semblable ou différent de $R_9$ ;

— un radical alcényle linéaire ou ramifié, contenant de 2 à 12 atomes de carbone, de préférence de 4 à 8 atomes de carbone, et tout particulièrement un radical alcényle dérivé du diène conjugué à carbonyler ;

— n représente un nombre entier supérieur ou égal à 1 et inférieur ou égal à 10 et de préférence inférieur ou égal à 6.

Comme indiqué ci-avant l'anion desdits sels d'onium pourra être choisi parmi : $PO_4^{3-}$, $HPO_4^{2-}$, $H_2PO_4^-$, $CH_3SO_3^-$, Ph-$SO_3^-$, (Ph représentant un radical phényle), $NO_3^-$, $SO_4^{2-}$, $PF_6^-$, $Cl^-$, $Br^-$.

On recourra avantageusement aux anions $Cl^-$ et $Br^-$ et plus particulièrement à l'anion $Cl^-$.

A titre d'exemple de cations onium quaternaire répondant à la formule (I) on peut citer les cations :

— tétraméthylammonium,
— triéthylméthylammonium,
— tributylméthylammonium,
— triméthylpropylammonium,
— tétraéthylammonium,
— tétrabutylammonium,
— dodécyltriméthylammonium,
— méthyltrioctylammonium,
— heptyltributylammonium,
— tétrapropylammonium,
— tétrapentylammonium,
— tétrahexylammonium,
— tétraheptylammonium,
— tétraoctylammonium,
— tétradécylammonium,

— butyltripropylammonium,
— méthyltributylammonium,
— pentyltributylammonium,
— méthyldiéthylpropylammonium,
— éthyldiméthylpropylammonium,
— tétradodécylammonium,
— tétraoctadécylammonium,
— hexadécyltriméthylammonium,
— benzyltriméthylammonium,
— benzyldiméthylpropylammonium,
— benzyldiméthyloctylammonium,
— benzyltributylammonium,
— benzyltriéthylammonium,
— phényltriméthylammonium,
— benzyldiméthyltétradécylammonium,
— benzyldiméthylhexadécylammonium,
— diméthyldiphénylammonium,
— méthyltriphénylammonium,
— butène-2 yltriéthylammonium,
— N,N-diméthyl-tétraméthylènammonium,
— N,N-diéthyl-tétraméthylènammonium,
— tétraméthylphosphonium,
— tétrabutylphosphonium,
— éthyltriméthylphosphonium,
— triméthylpentylphosphonium,
— octyltriméthylphosphonium,
— dodécyltriméthylphosphonium,
— triméthylphénylphosphonium,
— diéthyldiméthylphosphonium,
— dicyclohexyldiméthylphosphonium,
— diméthyldiphénylphosphonium,
— cyclohexyltriméthylphosphonium,
— triéthylméthylphosphonium,
— méthyltri(isopropyl)phosphonium,
— méthyltri(n-propyl)phosphonium,
— méthyltri(n-butyl)phosphonium,
— méthyltri(méthyl-2 propyl)phosphonium,
— méthyltricyclohexylphosphonium,
— méthyltriphénylphosphonium,
— méthyltribenzylphosphonium,
— méthyltri(méthyl-4 phényl)phosphonium,
— méthyltrixylylphosphonium,
— diéthylméthylphénylphosphonium,
— dibenzylméthylphénylphosphonium,
— éthyltriphénylphosphonium,
— tétraéthylphosphonium,
— éthyltri(n-propyl)phosphonium,
— triéthylpentylphosphonium,
— hexadécyltributylphosphonium,
— éthyltriphénylphosphonium,
— n-butyltri(n-propyl)phosphonium,
— butyltriphénylphosphonium,
— benzyltriphénylphosphonium,
— (β-phényléthyl)diméthylphénylphosphonium,
— tétraphénylphosphonium,
— triphényl(méthyl-4 phényl)phosphonium,
— tétrakis(hydroxyméthyl)phosphonium,
— tétrakis(hydroxy-2 éthyl)phosphonium,
— tétraphénylarsonium.

Parmi les cations répondant à la formule (II), on peut citer les cations :
— N-méthylpyridinium,
— N-éthylpyridinium,
— N-hexadécylpyridinium,
— N-méthylpicolinium.

Parmi les cations répondant à la formule (III), on peut citer les cations :

— bis(butène-2 yldiméthylammonium)-1,3 propane,
— bis(triméthylammonium)-1,2 éthane,
— bis(triméthylammonium)-1,3 propane,
— bis(triméthylammonium)-1,4 butane,
— bis(triméthylammonium)-1,3 butane.

Il peut être intéressant lorsque le sel d'onium choisi contient comme anion $Cl^-$ ou $Br^-$, et comme cation un cation de formule (I) ou (III) dans laquelle un des radicaux $R_1$ à $R_4$ ou le radical $R_{10}$ est un radical alcényle dérivant du diène conjugué mis en œuvre, de préparer le sel d'onium « in situ » au lieu de le préparer préalablement à l'opération de carbonylation.

En effet il est particulièrement aisé de préparer ce type de produit par action d'une amine tertiaire par exemple sur le ou les produits de la réaction du diène conjugué mis en œuvre et de l'acide chlorhydrique ou bromhydrique.

Ainsi lorsque le diène conjugué à carbonyler est du butadiène-1,3 on pourra utiliser comme sel d'onium quaternaire du chlorure ou du bromure de buténýl-trialkylammonium, qu'il sera possible d'obtenir « in situ » dans le milieu de carbonylation, par action d'une alkylamine tertiaire sur du chloro-1 ou bromo-1 butène-2 ou sur du chloro-3 ou bromo-3 butène-1 ; si l'amine tertiaire est la triéthylamine par exemple, on pourra ainsi préparer « in situ » un chlorure ou un bromure de buténÿltriéthylammonium.

Parmi les catalyseurs au palladium pouvant être mis en œuvre pour réaliser le procédé faisant l'objet de l'invention, on peut citer :

— le palladium métallique déposé sur un support, tel que le charbon, l'alumine, la silice...,
— les oxydes de palladium,
— les sels ou les complexes π-allyliques de palladium, dont l'anion coordiné au cation palladium est choisi parmi les anions suivants : carboxylates tels que formiate, acétate, propionate, benzoate ; $SO_4^{2-}$, $NO_3^-$, acétylacétonate, halogénures tels $Cl^-$, et $Br^-$ et, de préférence, $Cl^-$ ;
— ou les complexes de palladium zéro comprenant des ligands organiques ne contenant pas d'éléments du groupe VB, complexes tels que le bis(dibenzalacétone) palladium ou le bis(cyclooctadiène-1,5) palladium.

Le procédé faisant l'objet de l'invention est tout particulièrement intéressant pour la préparation d'acides carboxyliques β,γ-insaturés dérivés des diènes conjugués présentant dans leur molécule le squelette butadiène-1,3.

Parmi les diènes conjugués présentant dans leur molécule le squelette butadiène-1,3, on peut citer :

— les diènes aliphatiques linéaires ou ramifiés, contenant de 4 à 12 atomes de carbone, et de préférence de 4 à 8 atomes de carbone éventuellement substitués par des groupes inertes tels que : phényle, cyclohexyle, nitro et oxo ;
— les diènes cycliques contenant de 6 à 8 atomes de carbone.

Comme exemples concrets de diènes conjugués, on peut citer : le butadiène-1,3, l'isoprène, le piperylène, l'hexadiène-1,3, l'hexadiène-2,4, le chloroprène, le cyclohexyl-1 butadiène-1,3, le phényl-1 butadiène-1,3, l'octadiène-2,4, le méthyl-3 pentadiène-1,3, le méthyl-2 pentadiène-2,4, le cyclohexadiène-1,3, le cyclooctadiène-1,3.

L'opération de carbonylation est favorablement réalisée en présence d'acide chlorhydrique ou bromhydrique, et tout particulièrement en présence d'acide chlorhydrique.

L'acide chlorhydrique peut être introduit dans le milieu de carbonylation sous forme gazeuse, sous forme d'une solution aqueuse ou sous forme d'un composé organique susceptible de libérer de l'acide chlorhydrique dans le milieu, par exemple sous forme de chloro-1 butène-2 ou de chloro-3 butène-1 dans le cas de carbonylation du butadiène. Le choix parmi ces méthodes d'introduction sera avant tout guidé par des considérations d'ordre pratique.

Les quantités de réactifs à utiliser pour réaliser le procédé faisant l'objet de la présente invention peuvent varier entre des limites très larges ; il est évident que lesdites quantités seront choisies de manière à favoriser l'économie globale du procédé.

Ainsi, bien qu'il soit possible d'utiliser de 0,1 à 5 fois la quantité d'eau stœchiométriquement nécessaire, il est préférable afin d'éviter tout risque de précipitation du palladium sous forme de granules métalliques (précipitation due à une trop forte concentration en eau), de mettre en œuvre le procédé avec un rapport molaire eau/diène conjugué compris entre 0,5 et 2 environ et, de manière avantageuse, avec un rapport molaire eau/diène conjugué inférieur ou égal à 1.

De même, la bonne activité des catalyseurs au palladium permet l'emploi desdits catalyseurs en quantité très faible (correspondant à un rapport molaire diène conjugué/palladium de l'ordre de 2 500) ; l'emploi d'une quantité élevée de catalyseur (correspondant à un rapport molaire diène conjugué/palladium de l'ordre de 100) n'est pas nuisible ; le but recherché étant de réaliser une opération de carbonylation suffisamment rapide et sélective, sans dépenser trop de catalyseur, un rapport diène conjugué/palladium compris entre environ 150 et 2 000 et en particulier entre environ 200 et 1 500 est généralement préférable ; il a été constaté qu'un rapport molaire compris entre environ 500 et 700 conduit généralement à de bons résultats.

Il a été constaté que l'effet bénéfique apporté par la présence dans le milieu de carbonylation d'un sel d'onium quaternaire correspondant à la définition donnée ci-dessus est sensible à partir d'un rapport

molaire cation onium/palladium de 15 ; notamment un effet particulièrement intéressant a été constaté lorsque ledit rapport est compris entre 20 et 300, un rapport plus élevé, n'étant toutefois pas nocif pour la réaction de carbonylation.

Pour une bonne réalisation du procédé de carbonylation il sera préférable pour le choix du rapport cation onium/palladium de tenir compte de la concentration en palladium du milieu et notamment du rapport molaire diène conjugué/palladium ; ainsi plus le rapport diène conjugué/palladium est élevé, plus il y a intérêt à utiliser un rapport cation onium/palladium élevé.

La quantité minimale d'hydracide halogéné à utiliser dépend pour une large mesure de la nature de l'anion du sel d'onium quaternaire défini précédemment.

En effet lorsque l'anion du sel d'onium quaternaire est choisi parmi $Cl^-$ et $Br^-$, la quantité d'hydracide halogéné à utiliser correspond à un rapport molaire hydracide halogéné/palladium d'au moins 5. Toutefois, afin d'éviter tout risque de précipitation du palladium sous forme de granules métalliques (précipitation due à une concentration trop faible en hydracide du milieu) ou de réaction parasite entre l'hydracide et les produits de la réaction de carbonylation (due à une concentration trop importante du milieu en hydracide), un rapport molaire hydracide halogéné/palladium compris entre 10 et 150, et de préférence entre 20 et 100, pourra être favorablement choisi.

Par contre, lorsque l'anion du sel d'onium quaternaire utilisé est différent de $Cl^-$ et de $Br^-$, il convient d'utiliser une quantité supplémentaire d'hydracide halogéné qui correspond à la quantité de cations onium quaternaire ainsi introduite.

Toutefois, pour simplifier l'exposé de ce qui suit, dans le rapport molaire hydracide halogéné/palladium cette quantité supplémentaire n'est pas prise en compte mais elle est supposée avoir été ajoutée dans le cas où elle est nécessaire.

Les entités élémentaires correspondant aux termes « moles » sont les suivantes :

| | |
|---|---|
| — eau : | molécule-gramme |
| — diène conjugué : | molécule-gramme |
| — hydracide halogéné : | molécule-gramme |
| — palladium : | atome-gramme |
| — cation onium quaternaire : | ion-gramme. |

L'opération de carbonylation pourra généralement être réalisée à une température comprise entre 60 et 170 °C, de préférence entre 90 et 140 °C, sous une pression d'oxyde de carbone comprise entre 50 et 500 bars et, de préférence, comprise entre 80 et 300 bars.

La température et la pression sont choisies en fonction de l'activité et/ou de la sélectivité recherchée pour un catalyseur donné, de manière à obtenir les meilleurs résultats possibles. En effet, il est connu que si la température diminue, la vitesse de conversion du diène diminue mais la sélectivité en monoacide recherchée augmente. Il a également été constaté que si la pression d'oxyde de carbone augmente, la sélectivité en monoacide recherchée augmente.

Le procédé faisant l'objet de l'invention peut être réalisé en continu ou en discontinu ; on a constaté qu'on pouvait obtenir un taux de conversion du butadiène-1,3 acceptable en opérant en discontinu pendant 2 à 5 heures. Si ledit procédé est peu sensible à la présence de gaz inertes tels que azote, argon ou gaz carbonique qui peuvent être présents à côté de l'oxyde de carbone, il conviendra, toutefois, de veiller à ce que l'hydrogène susceptible d'être présent dans le monoxyde de carbone de qualité technique n'excède pas de 3 à 5 % en volume afin de limiter la réaction d'hydrogénation du diène conjugué.

Les exemples ci-après illustrent l'invention sans toutefois en limiter le domaine ou l'esprit.

Exemples 1 à 16 ; Essais témoins (a) et (b) :

Préparation de l'acide pentène-3 oïque à partir du butadiène :

Dans les exemples 1 à 16 et les tableaux ci-après, les conventions suivantes sont utilisées :

— Réf. : référence de l'exemple (ou de l'essai comparatif),

— mmol : millimole,

— BD : butadiène-1,3,

— $H^+$ : hydracide halogéné c'est-à-dire

— HCl engagé sous forme d'une solution aqueuse pour l'exemple 6,

— HBr engagé sous forme de bromo-1 butène-3 dans l'exemple 9,

— HCl engagé sous forme de chloro-1 butène-3 dans tous les autres exemples ou essais comparatifs,

— Pd : catalyseur au palladium engagé sous forme

— de bis[chloro π-allylpalladium(II)] dans les exemples 6 et 10,

— de bis(dibenzylacétone) palladium dans l'exemple 9,

— de $PdCl_2$ dans tous les autres exemples et essais témoins,

— $H_2O$/BD : rapport molaire de l'eau au butadiène,

— BD/Pd : rapport molaire du butadiène au palladium,

— $H^+/Pd$ : rapport molaire de l'hydracide halogéné au palladium,

— Additif : en principe, un sel d'onium dont la nature est précisée par recours à des formules « condensées » dans lesquelles

— Me désigne un radical méthyle,

— Bu désigne un radical n-butyle,

— Hex désigne un radical n-hexyle,

— Hep désigne un radical n-heptyle,

— Oct désigne un radical n-octyle,

— Additif/Pd : rapport molaire du sel d'onium au palladium,

— T : température en °C,

— P(CO) : pression du monoxyde de carbone en bars

— la présence du signe = devant la valeur de cette pression signifie que l'opération est réalisée à pression constante,

— l'absence de ce signe signifie que l'opération de carbonylation est réalisée à pression non constante, (ces deux modes opératoires sont précisés dans ce qui suit),

— t : durée de l'essai en température exprimée en heure,

— Stb : stabilité du palladium basée sur l'observation :

— d'une absence de précipitation du palladium notée par le signe +,

— d'une précipitation importante du palladium notée par le signe 0,

— TT : taux de conversion global du butadiène (en %),

— RT : nombre de moles du produit considéré pour 100 moles de butadiène transformées,

— A : activité du catalyseur exprimée en nombre de moles d'acide pentène-3 oïque obtenues par mole de palladium et par heure,

— $P_3$ : acide pentène-3 oïque,

— P' : acide méthyl-2 butène-3 oïque,

— PA : acides pentanoïque et méthyl-2 butanoïque,

— AC9 : acide nonadiène-3,8 oïque,

— DC6 : diacides à 6 atomes de carbone (majoritairement, l'acide méthyl-2 pentanedioïque-1,5),

— C4 : butènes,

— VCH : vinylcyclohexène,

— tr : traces.

Mode opératoire général

Dans un autoclave de 125 $cm^3$ en alliage nickel-molybdène de marque HASTELLOY B2, muni d'une tête amovible et d'une vanne, on introduit sous courant d'argon :

— le catalyseur au palladium,

— de l'eau dans laquelle on a, le cas échéant, solubilisé du gaz chlorhydrique,

— le cas échéant, du chloro-1 butène-3 ou du bromo-1 butène-3,

— le cas échéant, un additif.

La tête de l'autoclave est alors vissée ; on introduit alors dans l'autoclave, par l'intermédiaire de la vanne précitée, du butadiène. Du monoxyde de carbone est ensuite introduit. Cette introduction est effectuée différemment selon que l'opération est réalisée à pression constante ou à pression non constante.

Carbonylation à pression constante de CO

L'autoclave agité par secousses est porté à la température (T) avec alimentation à pression constante [P(CO)] de monoxyde de carbone gazeux de qualité technique contenant environ 0,8 % en volume d'hydrogène. On laisse la réaction de dérouler pendant le temps (t) à cette température.

Carbonylation à pression non constante de CO

On charge du monoxyde de carbone de qualité technique, contenant environ 0,8 % en volume d'hydrogène, — par exemple 120 bars (CNT), dans l'exemple 1 — et l'on porte l'autoclave à la température (T). La pression dans l'autoclave atteint la valeur indiquée par [P(CO)], par exemple 145 bars, dans l'exemple 1. On laisse la réaction se dérouler pendant le temps (t) à la température indiquée, en laissant baisser, peu à peu, la pression à l'intérieur de l'autoclave.

Après un temps (t) à la température indiquée, à pression constante ou non, l'autoclave est refroidi à 15 °C et dégazé lentement. La masse réactionnelle résultante est estérifiée à l'aide d'acétals en milieu acide, puis analysée par chromatographie en phase gazeuse. Les différents acides contenus par la masse réactionnelle sont donc analysés sous forme de leurs esters.

(On vérifie que la méthode est fiable, d'une part en s'assurant, par potentiométrie, qu'au moins 95 % des fonctions acides —COOH sont estérifiées et d'autre part, en soumettant, au préalable, à l'analyse des échantillons obtenus par estérification de chaque acide en cause et de mélanges connus des divers produits de la réaction).

On a en outre, lors du dégazage de l'autoclave, piégé les gaz dans de l'éthanol à – 78 °C puis dosé par chromatographie en phase gazeuse les butènes (produits d'hydrogénation du butadiène).

La nature et la quantité d'additif, ainsi que les quantités de réactifs et de catalyseur sont indiquées dans le tableau I ci-après. Les conditions particulières figurent également dans ce tableau.

Les résultats obtenus figurent dans le tableau II ci-après, dans lequel on a également rappelé la nature de l'additif utilisé.

— L'essai témoin (a) réalisé en l'absence d'additif, démontre qu'en l'absence d'additif, le catalyseur du palladium n'est pas stable, le rendement (RT) en acide pentène-3 oïque est très faible et la quantité de butènes formés est très importante.

— L'essai témoin (b) réalisé en présence d'un chlorure minéral (qui n'est pas un additif rentrant dans le cadre de la présente invention) démontre qu'en présence d'un chlorure minéral, le catalyseur au palladium n'est pas stable, le rendement (RT) en acide pentène-3 oïque n'est pas très bon (64,5 %).

— L'exemple 16 réalisé en présence d'un additif selon l'invention démontre l'intérêt de ce type d'additif puisqu'en la présence d'un tel additif le catalyseur au palladium est stable et le rendement (RT) en acide pentène-3 oïque atteint 82 %.

— Les exemples 1 à 4 illustrent la mise en œuvre de divers chlorures d'onium quaternaire. Le catalyseur au palladium est stable ; le rendement en acide pentène-3 oïque est de l'ordre de 75 à 85 %.

— Les exemples 5 à 8 illustrent la mise en œuvre du chlorure de tétrabutylphosphonium dans diverses conditions.

— L'exemple 9 montre que les bromures d'onium quaternaire sont moins actifs que les chlorures correspondants mais les bromures en cause permettent aussi de stabiliser le catalyseur au palladium et d'obtenir un rendement satisfaisant en acide recherché.

— Les exemples 10 à 13 illustrent diverses variations des conditions opératoires.

— Les exemples 14 à 15 montrent l'intérêt d'utiliser un faible rapport molaire $H_2O$/BD.

Dans l'ensemble de ces exemples, on détecte des traces de divers composés non mentionnés dans le tableau II à savoir :

— produits d'hydroformylation du butadiène (penténals, pentanals),

— produits de lactonisation des acides insaturés ($\gamma$ et $\delta$-valérolactones, 2-méthylbutyrolactone),

— acide chloropentanoïque : (RT) 7,2 % dans l'exemple 6, 4,9 % dans l'exemple 14 et moins de 3 % dans l'ensemble des autres exemples.

Exemple 17

Préparation de l'acide méthyl-4 pentène-3 oïque à partir de l'isoprène

Dans l'autoclave et selon le mode opératoire décrit précédemment, on réalise un essai sur une charge constituée par :

278 mmol d'isoprène,
278 mmol d'eau,
0,423 mmol de $PdCl_2$,
21,2 mmol de chloro-1 butène-3,
24,4 mmol de chlorure de tétrabutylphosphonium ($Bu_4P^+Cl^-$).

Les rapports molaires sont les suivants :
eau/isoprène : 1,00
isoprène/Pd : 657
HCl (provenant du chorobutène)/Pd : 50
$Bu_4P^+Cl^-$/Pd : 57,7.

Après deux heures de réaction à 100 °C sous 145 bars (pression non constante) le mélange réactionnel est analysé comme indiqué pour les exemples précédents.

Il renferme notamment les produits suivants :
135,5 mmol d'acide méthyl-4 pentène-3 oïque,
9,2 mmol d'acide diméthyl-2,3 butène-3 oïque,
29,3 mmol de produits d'hydrogénation des doubles liaisons C=C des acides ci-dessus (acide méthyl-4 pentanoïque et acide diméthyl-2,3 butanoïque).

(Voir Tableaux I et II pages 9 et 10)

Tableau I

| Réf | BD mmol | $H_2O$ mmol | $H^+$ mmol | Pd mmol | $\frac{H_2O}{BD}$ | $\frac{BD}{Pd}$ | $\frac{H^+}{Pd}$ | ADDITIF nature | ADDITIF mmol | $\frac{Additif}{Pd}$ | T | P(CO) | t |
|---|---|---|---|---|---|---|---|---|---|---|---|---|---|
| 1 | 278 | 278 | 21,2 | 0,43 | 1,00 | 657 | 50 | $Bu_4N^+Cl^-$ | 48,8 | 115 | 100 | 145 | 2 |
| 2 | " | " | " | " | " | " | " | $MeBu_3N^+Cl^-$ | " | " | " | " | " |
| 3 | 269 | " | " | " | 1,03 | 634 | " | $MeOct_3N^+Cl^-$ | " | " | " | " | " |
| 4 | 278 | " | " | " | 1,00 | 657 | " | $Hex_4N^+Cl^-$ | 24,4 | 58 | " | =145 | " |
| 5 | 250 | " | 50,0 | 1,00 | 0,90 | 250 | " | $Bu_4P^+Cl^-$ | 50,0 | 50 | 90 | 145 | " |
| 6 | 278 | " | 64,0 | 0,43 | 1,00 | 657 | 150 | " | 21,2 | 50 | 120 | 120 | " |
| 7 | " | " | 21,2 | " | " | " | 50 | " | 24,4 | 58 | 100 | =200 | " |
| 8 | " | " | " | " | " | " | " | " | " | " | " | = 80 | " |
| 9 | 315 | " | " | " | 0,88 | 745 | " | $Hep_4N^+Br^-$ | 16,3 | 38 | " | 145 | " |
| 10 | 278 | " | " | " | 1,00 | 657 | " | $Bu_4N^+Cl^-$ | 48,8 | 115 | " | 145 | " |
| 11 | " | " | " | " | " | " | " | $Bu_4P^+Cl^-$ | 24,4 | 58 | 120 | =145 | " |
| 12 | " | " | " | 0,22 | " | 1264 | 100 | " | " | 115 | 100 | =145 | " |
| 13 | " | " | " | 1,27 | " | 219 | 17 | " | " | 20 | " | =145 | " |
| 14 | " | 139 | " | 0,43 | 0,50 | 657 | 50 | " | 48,8 | 115 | " | =145 | " |
| 15 | " | 555 | " | " | 2,00 | " | " | $Me_4N^+Cl^-$ | 127,3 | 300 | 120 | =145 | 5 |
| 16 | 343 | 329 | 26,7 | 0,535 | 0,96 | 641 | " | $Bu_4P^+Cl^-$ | 10,7 | 20 | 90 | =145 | 3 |
| a | 315 | 328 | " | " | 1,04 | 588 | " | - | 0 | 0 | " | =145 | " |
| b | 352 | 329 | " | " | 0,94 | 658 | " | $Cs^+Cl^-$ | 10,7 | 20 | " | =145 | " |

Tableau II

| Réf | ADDITIF (nature) | Stb | TT | A | RT P3 | RT P' | RT PA | RT AC9 | RT DC6 | RT C4 | RT VCH |
|---|---|---|---|---|---|---|---|---|---|---|---|
| 1 | $Bu_4N^+Cl^-$ | + | 60,4 | 153 | 77,2 | 0,8 | 3,7 | tr | 4,5 | 13,5 | tr |
| 2 | $MeBu_3N^+Cl^-$ | + | 23,5 | 59 | 76,1 | tr | 2,3 | " | 5,7 | 15,3 | " |
| 3 | $MeOct_3N^+Cl^-$ | + | 70,3 | 163 | 72,8 | 1,3 | 2,9 | 3,2 | 13,0 | 3,3 | 2,7 |
| 4 | $Hex_4N^+Cl^-$ | + | 59,6 | 165 | 84,5 | 1,6 | 1,3 | 1,9 | 3,8 | 5,9 | tr |
| 5 | $Bu_4P^+Cl^-$ | + | 34,8 | 32 | 72,5 | 0,1 | 7,4 | 0,2 | 10,8 | 7 | " |
| 6 | " | + | 29,4 | 41 | 42,5 | 0,3 | 8,4 | 7,2 | 1,9 | 18,9 | 6,8 |
| 7 | " | + | 47,9 | 136 | 86,5 | 1,4 | 1,6 | 0,2 | 3,5 | 5,0 | 0,6 |
| 8 | " | + | 19,1 | 49 | 78,0 | 1,6 | 0,6 | 3,2 | 1,9 | 7,5 | 2,7 |
| 9 | $Hep_4N^+Br^-$ | + | 9,4 | 29 | 82,7 | 1,7 | 5,1 | tr | 4,4 | tr | 6,1 |
| 10 | $Bu_4N^+Cl^-$ | + | 67,5 | 179 | 80,6 | 0,8 | 3,4 | 1,2 | 7,6 | 5,6 | tr |
| 11 | $Bu_4P^+Cl^-$ | + | 54,4 | 113 | 63,0 | 0,5 | 6,6 | 0,5 | 4 | 20,5 | " |
| 12 | " | + | 26,1 | 104 | 62,7 | 0,9 | 0,4 | tr | 1,8 | 31,2 | 0,6 |
| 13 | " | + | 56,6 | 38 | 61,0 | 0,7 | 11,0 | 0,4 | 8,6 | 15,0 | tr |
| 14 | " | + | 53,2 | 135 | 77,0 | 0,8 | 1,6 | 0,9 | 3,3 | 10,2 | 1,4 |
| 15 | $Me_4N^+Cl^-$ | + | 24,7 | 19 | 58,8 | 0,5 | 2,3 | 11,1 | 0,9 | 10,5 | 11,5 |
| 16 | $Bu_4P^+Cl^-$ | + | 29,4 | 52 | 82,2 | 1,8 | 0,3 | 3,2 | 1,3 | 8,4 | 1,6 |
| a | - | 0 | 15,5 | 3 | 5,4 | 0,6 | 1,6 | 0,2 | tr | 89 | 2,9 |
| b | $Cs^+Cl^-$ | 0 | 18 | 25 | 64,5 | " | 0,4 | 19,9 | 0,4 | 9 | 2,2 |

**Revendications**

1. Procédé de préparation d'acides carboxyliques β,γ-insaturés, par carbonylation d'un diène conjugué en présence d'eau, d'un hydracide halogéné et d'un catalyseur au palladium, à une température supérieure à 60 °C et sous une pression totale (en température) supérieure à 50 bars, caractérisé en ce que :

a) l'opération de carbonylation est réalisée en outre en présence d'un sel d'onium quaternaire d'un élément du groupe VB choisi parmi l'azote, le phosphore et l'arsenic, ledit élément étant quadricoordiné à des atomes de carbone et ledit sel présentant un anion choisi parmi les bases « dures » ou « intermédiaires » du groupe constitué par les anions : $PO_4^{3-}$, $HPO_4^{2-}$, $H_2PO_4^-$, $CH_3SO_3^-$, $Ph\text{-}SO_3^-$, (Ph représentant un radical phényle), $NO_3^-$, $SO_4^{2-}$, $PF_6^-$, $Cl^-$, ou $Br^-$,

b) le catalyseur au palladium est constitué par :

— du palladium métallique éventuellement déposé sur un support,

— un oxyde de palladium,

— un sel ou un complexe π-allylique de palladium dont l'anion coordiné au cation palladium est une base « dure » ou « intermédiaire » du groupe constitué par les anions carboxylates, $SO_4^{2-}$, $NO_3^-$, acétylacétonate, chlorure et bromure, ou

— un complexe du palladium zéro comprenant des ligands organiques ne contenant pas d'éléments du groupe VB.

2. Procédé selon la revendication 1, caractérisé en ce que le sel d'onium quaternaire présente un cation onium quaternaire répondant à l'une des formules (I) à (III) suivantes :

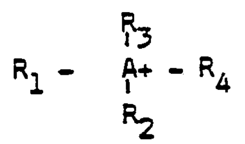

(I)

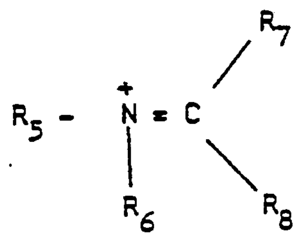

(II)

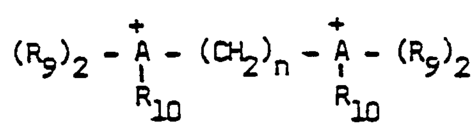

(III)

dans lesquelles :

— A représente un atome d'azote, de phosphore ou d'arsenic,

— $R_1$, $R_2$, $R_3$, $R_4$ sont identiques ou différents et représentent :

— un radical alkyle linéaire ou ramifié contenant de 1 à 16 atomes de carbone, éventuellement substitué par un groupe phényle, hydroxy, halogéno, nitro, alcoxy ou alcoxycarbonyle, le groupement alcoxy renfermant au maximum 4 atomes de carbone ;

— un radical alcényle linéaire ou ramifié contenant de 2 à 12 atomes de carbone ;

— un radical aryle contenant de 6 à 10 atomes de carbone, éventuellement substitué par un ou des radicaux alkyles contenant de 1 à 4 atomes de carbone, alcoxy, alcoxycarbonyle, le groupement alcoxy renfermant au maximum 4 atomes de carbone ou halogéno ;

— deux desdits radicaux $R_1$ à $R_4$ pouvant former ensemble un radical alkylène, alcénylène ou alcadiénylène, linéaire ou ramifié, contenant de 3 à 6 atomes de carbone ;

— $R_5$, $R_6$, $R_7$, $R_8$ sont identiques ou différents et représentent :

— un radical alkyle linéaire ou ramifié contenant de 1 à 4 atomes de carbone ;

— les radicaux $R_7$ et $R_8$ pouvant former ensemble un radical alkylène contenant de 3 à 6 atomes de carbone ;

— les radicaux $R_6$ et $R_7$ ou $R_6$ et $R_8$ pouvant former ensemble un radical alkylène, alcénylène ou alcadiénylène, contenant 4 atomes de carbone et constituant avec l'atome d'azote un hétérocycle azoté ;

— $R_9$ représente un radical alkyle linéaire ou ramifié contenant de 1 à 4 atomes de carbone ou un radical phényle ;

— $R_{10}$ représente :

— un radical alkyle linéaire ou ramifié contenant de 1 à 4 atomes de carbone, semblable ou différent de $R_9$ ;

— un radical alcényle linéaire ou ramifié, contenant de 2 à 12 atomes de carbone ;

— n représente un nombre entier supérieur ou égal à 1 et inférieur ou égal à 10.

3. Procédé selon la revendication 2, caractérisé en ce que lorsqu'ils représentent des radicaux alcényles, les groupes $R_2$, et $R_{10}$ contiennent de 4 à 8 atomes de carbone.

4. Procédé selon la revendication 3, caractérisé en ce que les radicaux $R_2$ et $R_{10}$ sont des radicaux alcényles dérivés du diène conjugué mis en œuvre.

5. Procédé selon la revendication 2, caractérisé en ce que n est un nombre entier inférieur ou égal à 6.

6. Procédé selon l'une quelconque des revendications 2 à 5, caractérisé en ce que ledit cation est un cation :
— tétraméthylammonium,
— tétrabutylammonium,
— tétrahexylammonium,
— tétraheptylammonium,
— méthyltributylammonium,
— méthyltrioctylammonium, ou
— tétrabutylphosphonium.

7. Procédé selon la revendication 1, caractérisé en ce que le catalyseur au palladium est un sel de palladium ou un complexe π-allylique de palladium dont l'anion coordiné au cation palladium est un anion carboxylate, $SO_4^{2-}$, $NO_3^-$, acétylacétonate, chlorure ou bromure ou un complexe de palladium zéro comprenant des ligands organiques ne contenant pas d'éléments du groupe VB.

8. Procédé selon la revendication 1, caractérisé en ce que l'opération de carbonylation est réalisée en présence de quantités de réactifs correspondant aux rapports molaires suivants :

| | |
|---|---|
| — eau/diène conjugué : | de 0,1 à 5 |
| — diène conjugué/palladium : | de 100 à 2 500 |
| — cation onium/palladium : | au moins 15 |
| — hydracide halogéné/palladium : | au moins 5. |

9. Procédé selon la revendication 8, caractérisé en ce que lesdits rapports molaires sont les suivants :

| | |
|---|---|
| — eau/diène conjugué : | de 0,5 à 2 |
| — diène conjugué/palladium : | de 200 à 1 500 |
| — cation onium/palladium : | de 20 à 300 |
| — hydracide halogéné/palladium : | de 10 à 150. |

10. Procédé selon la revendication 9, caractérisé en ce que le rapport molaire hydracide halogéné/palladium est compris entre 20 et 100.

11. Procédé de préparation de l'acide pentène-3 oïque par carbonylation du butadiène en présence d'eau, d'acide chlorhydrique et d'un catalyseur au palladium, à une température comprise entre 60 et 170 °C et sous une pression d'oxyde de carbone de 50 à 500 bars, caractérisé en ce que :

a) le milieu de carbonylation contient en outre un sel d'onium quaternaire d'un élément du groupe VB choisi parmi l'azote, le phosphore et l'arsenic, ledit élément étant quadricoordiné à des atomes de carbone et ledit sel présentant un anion choisi parmi les bases « dures » ou « intermédiaires » du groupe constitué par les anions : $PO_4^{3-}$, $HPO_4^{2-}$, $H_2PO_4^-$, $CH_3SO_3^-$, Ph-$SO_3^-$, (Ph représentant un radical phényle), $NO_3^-$, $SO_4^{2-}$, $PF_6^-$, $Cl^-$, ou $Br^-$,

b) le catalyseur au palladium est du chlorure de palladium II ou du bis [chlorure de π-allylpalladium (II)],

c) les différents réactifs sont mis en œuvre selon les rapports molaires suivants :
— eau/butadiène : de 0,5 à 2,
— butadiène/palladium : de 200 à 1 500,
— cation onium/palladium : de 20 à 300,
— acide chlorhydrique/palladium : de 10 à 150.

12. Procédé selon la revendication 11, caractérisé en ce que la température de réaction est comprise entre 90 et 140 °C et la pression d'oxyde de carbone est comprise entre 80 et 300 bars.

**Claims**

1. Process for the preparation of β,γ-unsaturated carboxylic acids, by carbonylation of a conjugated diene in the presence of water, a halogenated hydrogen acid and a palladium catalyst, at a temperature above 60 °C and at a total pressure (at temperature) above 50 bars, characterised in that :

a) the carbonylation operation is additionally carried out in the presence of a quaternary onium salt of a group VB element chosen from nitrogen, phosphorus and arsenic, the said element being quadricoordinated to carbon atoms and the said salt containing an anion chosen from the « hard » or « intermediate » bases of the group consisting of the anions : $PO_4^{3-}$, $HPO_4^{2-}$, $H_2PO_4^-$, $CH_3SO_3^-$, Ph-$SO_3^-$, (Ph denoting a phenyl radical), $NO_3^-$, $SO_4^{2-}$, $PF_6^-$, $Cl^-$ or $Br^-$,

b) the palladium catalyst consists of :
— metallic palladium optionally deposited on a support,
— a palladium oxide,
— a salt or a π-allyl complex of palladium in which the anion coordinated to the palladium cation is

a « hard » or « intermediate » base of the group consisting of carboxylate, $SO_4^{2-}$, $NO_3^-$, acetylacetonate, chloride and bromide anions, or

— a palladium zero complex incorporating organic ligands not containing group VB elements.

2. Process according to Claim 1, characterised in that the quaternary onium salt has a quaternary onium cation corresponding to one of the following formulae (I) to (III) :

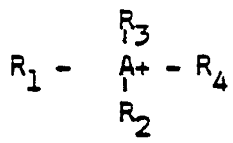

(I)

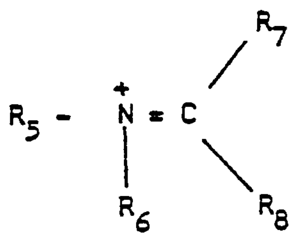

(II)

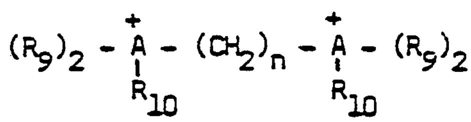

(III)

in which :

— A denotes a nitrogen, phosphorus or arsenic atom,

— $R_1$, $R_2$, $R_3$ and $R_4$ are identical or different and denote :

— a straight-chain or branched alkyl radical containing from 1 to 16 carbon atoms, optionally substituted by a phenyl, hydroxy, halogen, nitro, alkoxy or alkoxycarbonyl group, the alkoxy group containing at most 4 carbon atoms ;

— a straight-chain or branched alkenyl radical containing from 2 to 12 carbon atoms ;

— an aryl radical containing from 6 to 10 carbon atoms, optionally substituted by one or more alkyl radicals containing from 1 to 4 carbon atoms, or alkoxy or alkoxycarbonyl radicals, the alkoxy group containing at most 4 carbon atoms, or halogen radicals ;

— two of the said radicals $R_1$ to $R_4$ being capable of together forming a straight-chain or branched alkylene, alkenylene or alkadienylene radical containing from 3 to 6 carbon atoms ;

— $R_5$, $R_6$, $R_7$ and $R_8$ are identical or different and denote :

— a straight-chain or branched alkyl radical containing from 1 to 4 carbon atoms ;

— the radicals $R_7$ and $R_8$ being able together to form an alkylene radical containing from 3 to 6 carbon atoms ;

— the radicals $R_6$ and $R_7$ or $R_6$ and $R_8$ being able together to form an alkylene, alkenylene or alkadienylene radical containing 4 carbon atoms and forming a nitrogenous heterocyclic ring with the nitrogen atom ;

— $R_9$ denotes a straight-chain or branched alkyl radical containing from 1 to 4 carbon atoms or a phenyl radical ;

— $R_{10}$ denotes :

— a straight-chain or branched alkyl radical containing from 1 to 4 carbon atoms, similar to or different from $R_9$ ;

— a straight-chain or branched alkenyl radical containing from 2 to 12 carbon atoms ;

— n denotes an integer greater than or equal to 1 and smaller than or equal to 10.

3. Process according to Claim 2, characterised in that, when they denote alkenyl radicals, the groups $R_2$ and $R_{10}$ contain from 4 to 8 carbon atoms.

4. Process according to Claim 3, characterised in that the radicals $R_2$ and $R_{10}$ are alkenyl radicals derived from the conjugated diene employed.

5. Process according to Claim 2, characterised in that n is an integer smaller than or equal to 6.

6. Process according to any one of Claims 2 to 5, characterised in that the said cation is a :

— tetramethylammonium,
— tetrabutylammonium,
— tetrahexylammonium,
— tetraheptylammonium,
— methyltributylammonium,
— methyltrioctylammonium, or
— tetrabutylphosphonium cation.

7. Process according to Claim 1, characterised in that tne palladium catalyst is a palladium salt or a π-allyl complex of palladium in which the anion coordinated to the palladium cation is a carboxylate, $SO_4^{2-}$, $NO_3^-$, acetylacetonate, chloride or bromide anion or a palladium zero complex incorporating organic ligands not containing group VB elements.

8. Process according to Claim 1, characterised in that the carbonylation operation is carried out in the presence of quantities of reactants corresponding to the following molar ratios :
— water/conjugated diene : from 0.1 to 5
— conjugated diene/palladium : from 100 to 2,500
— onium cation/palladium : at least 15
— halogenated hydrogen acid/palladium : at least 5.

9. Process according to Claim 8, characterised in that the said molar ratios are the following :
— water/conjugated diene : from 0.5 to 2
— conjugated diene/palladium : from 200 to 1,500
— onium cation/palladium : from 20 to 300
— halogenated hydrogen acid/palladium : from 10 to 150.

10. Process according to Claim 9, characterised in that the molar ratio halogenated hydrogen acid/palladium is between 20 and 100.

11. Process for the preparation of penten-3-oic acid by carbonylation of butadiene in the presence of water, hydrochloric acid and a palladium catalyst, at a temperature between 60 and 170 °C and at a carbon monoxide pressure of 50 to 500 bars, characterised in that :

a) the carbonylation medium additionally contains a quaternary onium salt of a group VB element chosen from nitrogen, phosphorus and arsenic, the said element being quadricoordinated to carbon atoms and the said salt containing an anion chosen from the « hard » or « intermediate » bases of the group consisting of the anions : $PO_4^{3-}$, $HPO_4^{2-}$, $H_2PO_4^-$, $CH_3SO_3^-$, Ph-$SO_3^-$, (Ph denoting a phenyl radical), $NO_3^-$, $SO_4^{2-}$, $PF_6^-$, $Cl^-$, or $Br^-$,

b) the palladium catalyst is palladium (II) chloride or bis (π-allyl-palladium (II) chloride),

c) the various reactants are employed according to the following molar ratios :
— water/butadiene : from 0.5 to 2,
— butadiene/palladium : from 200 to 1,500,
— onium cation/palladium : from 20 to 300,
— hydrochloric acid/palladium : from 10 to 150.

12. Process according to Claim 11, characterised in that the reaction temperature is between 90 and 140 °C and the carbon monoxide pressure is between 80 and 300 bars.

**Patentansprüche**

1. Verfahren zur Herstellung von β,γ-ungesättigten Carbonsäuren durch Carbonylieren eines konjugierten Diens in Gegenwart von Wasser, einer Halogenwasserestoffsäure und eines Palladiumkatalysators bei einer Temperatur oberhalb 60 °C und unter einem Gesamtdruck (bei der Temperatur) über 50 bar, dadurch gekennzeichnet, daß :

a) die Carbonylierung zusätzlich in Gegenwart eines quaternären Oniumsalzes eines Elementes der Gruppe VB, ausgewählt unter Stickstoff, Phosphor und Arsen, durchgeführt wird, wobei dieses Element vierfach Kohlenstoffatomen zugeordnet (koordinativ an 4 Kohlenstoffatome gebunden) ist und das Salz ein Anion aufweist, das ausgewählt ist unter den « harten » oder « intermediären » Basen der Gruppe bestehend aus den Anionen $PO_4^{3-}$, $HPO_4^{2-}$, $H_2PO_4^-$, $CH_3SO_3^-$, Ph-$SO_3^-$, (wobei Ph eine Phenylgruppe bedeutet), $NO_3^-$, $SO_4^{2-}$, $PF_6^-$, $Cl^-$, oder $Br^-$,

b) der Palladiumkatalysator besteht aus :
— metallischem Palladium, gegebenenfalls abgeschieden auf einen Träger,
— einem Palladiumoxid,
— einem Salz oder einem π-Allylkomplex von Palladium, dessen dem Palladiumkation zugeordnetes Anion eine « harte » oder « intermediäre » Base der Gruppe bestehend aus dem Carboxylat-, $SO_4^{2-}$, $NO_3^-$, Acetylacetonat-, Chlorid- und Bromidanion ist, oder
— ein Komplex von nullwertigem Palladium, umfassend organische Liganden, die keine Elemente der Gruppe VB enthalten.

2. Verfahren nach Anspruch 1, dadurch gekennzeichnet, daß das quaternäre Oniumsalz ein quaternäres Oniumkation aufweist, das einer der folgenden Formeln (I) bis (III) entspricht

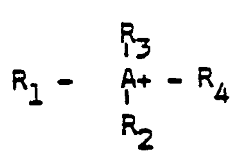

(I)

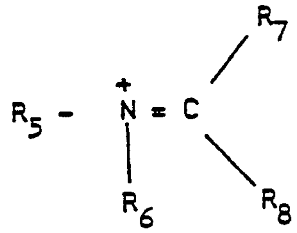

(II)

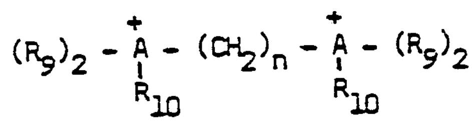

(III)

in denen :

— A ein Stickstoff-, Phosphor- oder Arsenatom bedeutet,

— $R_1$, $R_2$, $R_3$ und $R_4$ gleich oder verschieden sind und (jeweils) bedeuten :

— eine lineare oder verzweigte Alkylgruppe mit 1 bis 16 Kohlenstoffatomen, gegebenenfalls substituiert durch eine Phenyl-, Hydroxy-, Halogen-, Nitro-, Alkoxy- oder Alkoxycarbonylgruppe, wobei die Alkoxygruppe maximal 4 Kohlenstoffatome enthält ;

— eine lineare oder verzweigte Alkenylgruppe mit 2 bis 12 Kohlenstoffatomen ;

— eine Arylgruppe mit 6 bis 10 Kohlenstoffatomen, gegebenenfalls substituiert durch eine oder mehrere Alkylgruppe(n) mit 1 bis 4 Kohlenstoffatomen, Alkoxy-, oder Alkoxycarbonylgruppe(n), wobei die Alkoxygruppe maximal 4 Kohlenstoffatome enthält, oder Halogen ;

— zwei der Gruppen $R_1$ bis $R_4$ zusammen eine lineare oder verzweigte Alkylen-, Alkenylen- oder Alkadienylengruppe mit 3 bis 6 Kohlenstoffatomen bilden können ;

— $R_5$, $R_6$, $R_7$ und $R_8$ gleich oder verschieden sind und (jeweils) bedeuten :

— eine lineare oder verzweigte Alkylgruppe mit 1 bis 4 Kohlenstoffatomen ;

— die Gruppen $R_7$ und $R_8$ zusammen eine Alkylengruppe mit 3 bis 6 Kohlenstoffatomen bilden können ;

— die Gruppen $R_6$ und $R_7$ oder $R_6$ und $R_8$ zusammen eine Alkylen-, Alkenylen- oder Alcadienylgruppe bilden können, die 4 Kohlenstoffatome enthält und mit dem Stickstoffatom einen stickstoffhaltigen Heterocyclus bildet ;

— $R_9$ für eine lineare oder verzweigte Alkylgruppe mit 1 bis 4 Kohlenstoffatomen oder für eine Phenylgruppe steht ;

— $R_{10}$ bedeutet :

— eine lineare oder verzweigte Alkylgruppe mit 1 bis 4 Kohlenstoffatomen, die gleich ist wie oder verschieden von $R_9$ ;

— eine lineare oder verzweigte Alkenylgruppe mit 2 bis 12 Kohlenstoffatomen ;

— n eine ganze Zahl $\geqslant 1$ und $\leqslant 10$ ist.

3. Verfahren nach Anspruch 2, dadurch gekennzeichnet, daß die Gruppen $R_2$ und $R_{10}$, wenn sie Alkenylgruppen bedeuten, 4 bis 8 Kohlenstoffatome enthalten.

4. Verfahren nach Anspruch 3, dadurch gekennzeichnet, daß die Gruppen $R_2$ und $R_{10}$ von dem eingesetzten konjugierten Dien abgeleitete Alkenylgruppen sind.

5. Verfahren nach Anspruch 2, dadurch gekennzeichnet, daß n eine ganze Zahl $\leqslant 6$ ist.

6. Verfahren nach einem der Ansprüche 2 bis 5, dadurch gekennzeichnet, daß das Kation eines der folgenden Kationen ist :

— Tetramethylammonium,
— Tetrabutylammonium,
— Tetrahexylammonium,
— Tetraheptylammonium,
— Methyltributylammonium,
— Methyltrioctylammonium oder
— Tetrabutylphosphonium.

7. Verfahren nach Anspruch 1, dadurch gekennzeichnet, daß der Palladiumkatalysator ein Palladiumsalz oder ein π-Allylkomplex von Palladium ist, dessen dem Palladiumkation zugeordnetes Anion ein Carboxylatanion, $SO_4^{2-}$, $NO_3^-$, Acetylacetonat-, Chlorid- oder Bromidanion ist oder ein Komplex von nullwertigem Palladium, umfassend organische Liganden, die kein Element der Gruppe VB enthalten.

8. Verfahren nach Anspruch 1, dadurch gekennzeichnet, daß die Carbonylierung in Gegenwart von Mengen an Reaktionspartnern durchgeführt wird, die folgenden Molverhältnissen entsprechen :

— Wasser/konjugiertes Dien 0,1 bis 5
— konjugiertes Dien/Palladium 100 bis 2 500
— Oniumkation/Palladium mindestens 15
— Halogenwasserstoffsäure/Palladium mindestens 5.

9. Verfahren nach Anspruch 8, dadurch gekennzeichnet, daß die Molverhältnisse folgendermaßen lauten :

— Wasser/konjugiertes Dien 0,5 bis 2
— konjugiertes Dien/Palladium 200 bis 1 500
— Oniumkation/Palladium 20 bis 300
— Halogenwasserstoffsäure/Palladium 10 bis 150.

10. Verfahren nach Anspruch 9, dadurch gekennzeichnet, daß das Molverhältnis von Halogenwasserstoffsäure zu Palladium 20 bis 100 beträgt.

11. Verfahren zur Herstellung der Pent-3-ensäure durch Carbonylieren von Butadien in Gegenwart von Wasser, Chlorwasserstoff und einem Palladiumkatalysator bei einer Temperatur im Bereich von 60 bis 170 °C und unter einem Kohlenoxiddruck von 50 bis 500 bar, dadurch gekennzeichnet, daß :

a) das Carbonylierungsmedium zusätzlich ein quaternäres Oniumsalz eines Elementes der Gruppe VB enthält, das unter Stickstoff, Phosphor und Arsen ausgewählt ist, wobei das Element vierfach Kohlenstoffatomen zugeordnet (koordinativ an 4 Kohlenstoffatome gebunden) ist und das Salz ein Anion aufweist, das unter den « harten » oder « intermediären » Basen der Gruppe bestehend aus folgenden Anionen ausgewählt ist : $PO_4^{3-}$, $HPO_4^{2-}$, $H_2PO_4^-$, $CH_3SO_3^-$, $Ph\text{-}SO_3^-$, (wobei Ph eine Phenylgruppe bedeutet), $NO_3^-$, $SO_4^{2-}$, $PF_6^-$, $Cl^-$ oder $Br^-$ ;

b) der Palladiumkatalysator Palladium (II)-chlorid oder Bis [π-allyl-palladium (II)-chlorid] ist,

c) die verschiedenen Reaktionspartner entsprechend folgenden Molverhältnissen eingesetzt werden :

— Wasser/Butadien 0,5 bis 2
— Butadien/Palladium 200 bis 1 500
— Oniumkation/Palladium 20 bis 300
— Chlorwasserstoff/Palladium 10 bis 150.

12. Verfahren nach Anspruch 11, dadurch gekennzeichnet, daß die Reaktionstemperatur 90 bis 140 °C und der Kohlenoxiddruck 80 bis 300 bar beträgt.